# EUROPEAN PATENT APPLICATION

(11) **EP 1 983 355 A1**
(43) Date of publication of application: **22.10.2008**
(21) Application number: 08007348.9
(22) Date of filing: 15.04.2008
(51) Int. Cl.: G01S 15/89, G10K 11/32, G01N 29/22, B06B 1/06, A61B 8/00

(54) **Ultrasound probe having a transducer array with multiple radii of curvature**

(30) Priority: 16.04.2007 KR 20070037009
(71) Applicant: MEDISON CO., LTD., Kangwon-do 250-870 (KR)
(72) Inventor: Kye, Sang Bum, Gangnam-gu Seoul 135-280 (KR); Pu, You Chun, Gangnam-gu Seoul 135-280 (KR)
(74) Representative: Lorenz, Markus

(57) **Abstract**

The present invention relates to an ultrasound probe (40) used in an ultrasound system. The ultrasound probe includes a body (420) and a transmission/reception unit (410) mounted on the body and having at least one partially curved array transducer (510) operable to reciprocally convert ultrasound signals and electric signals. The curved array transducer may be sized and configured to have multiple radii of curvature.

## Description

The present application claims priority from Korean Patent Application No. 10-2007-0037009 filed on April 16, 2007, the entire subject matter of which is incorporated herein by reference.

### BACKGROUND OF THE INVENTION

### [Technical Field]

The present invention generally relates to ultrasound probes, and more particularly to an ultrasound probe having a transducer array with multiple radii of curvature.

### [Background Art]

The ultrasound system has become an important and popular diagnostic tool due to its non-invasive and non-destructive nature. Modern high-performance ultrasound imaging diagnostic systems and techniques are commonly used to produce two- or three-dimensional images of internal features of patients.

An ultrasound system generally uses a probe containing an array of piezoelectric elements to transmit and receive ultrasound signals. The ultrasound system forms an image of human internal tissues by electrically exciting transducer elements to generate ultrasound signals that travel into the body. Echoes reflected from tissues and organs return to the transducer element and are converted into electrical signals, which are amplified and processed to produce ultrasound data. The ultrasound data may include volume data obtained by using a 3-dimensional probe, etc.

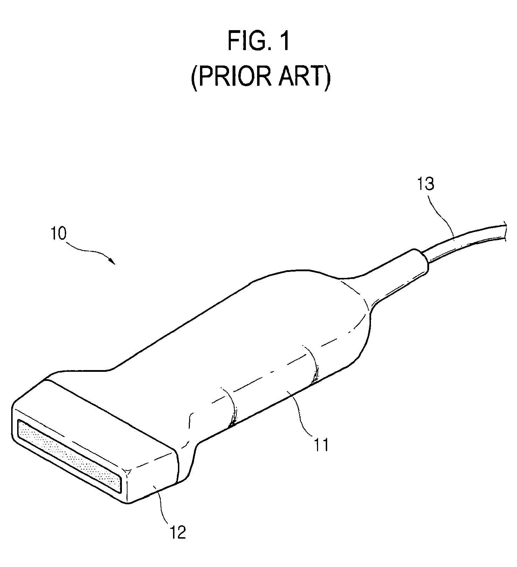

FIG. 1 is a schematic diagram showing a conventional ultrasound probe adopted in the ultrasound system. The ultrasound probe 10 may include a body 11 and an ultrasound transmission/reception (T/R) unit 12. The T/R unit 12 may be operable to transmit ultrasound signals to a target object and receive ultrasound echo signals reflected from the target object. The T/R unit 12 may include a transducer for reciprocally converting ultrasound signals and electrical signals.

Generally, the ultrasound system is used to perform examination and diagnosis of a patient through an ultrasound image. Thus, resolution of the ultrasound image provided from the ultrasound system is very important. In order to obtain high resolution of ultrasound image, an array transducer constructed with a plurality of transducer elements is adopted.

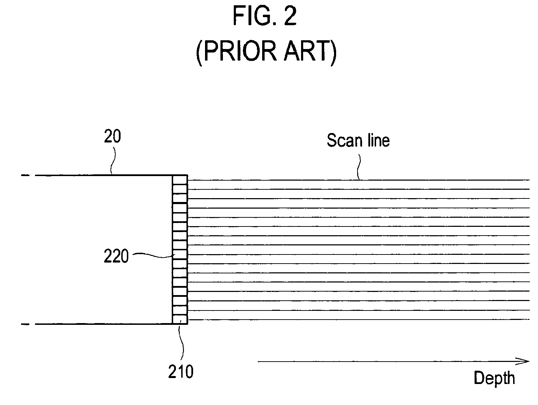

FIG. 2 is a schematic diagram showing scan lines set with respect to a linear array transducer. The linear array transducer 210 may include a plurality of transducer elements 220, e.g., 128 or 192 transducer elements. When a probe 20 containing the linear array transducer 210 (hereinafter referred to as a linear probe) is used to scan a target object, a plurality of scan lines are set normal to the respective transducer elements toward a depth direction, as shown in FIG. 2. In such a case, the scan lines are uniformly set in parallel with each other so that high resolution of an ultrasound image can be obtained in a depth direction while a viewing angle is relatively narrow.

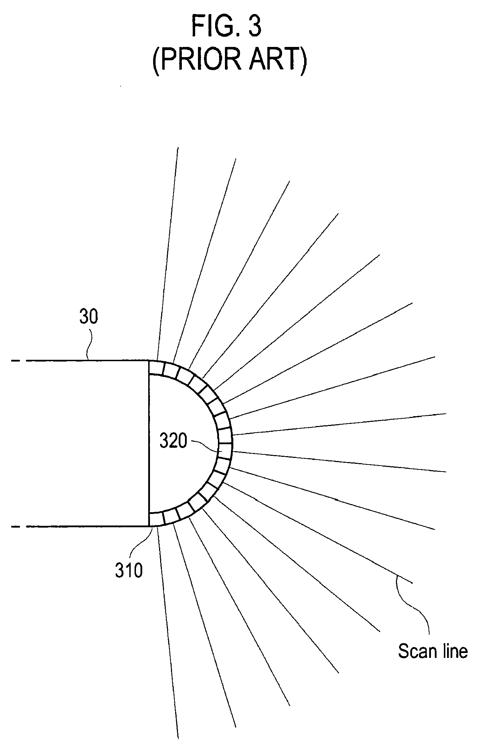

In order to widen the viewing angle of the ultrasound image, a probe having a convex type of array transducer (hereinafter referred to as a convex probe) has been adopted. As illustrated in FIG. 3, the convex probe 30 includes an array transducer 310, which is designed with a plurality of transducer elements aligned to a specific curvature. With the convex probe, scan lines are set normal to the respective transducer elements such that the set scan lines form a radial shape. Thus, a wide viewing angle of ultrasound image may be obtained. However, intervals between the scan lines become wider as the depth gets deeper, which lowers resolution of the ultrasound image.

Further, a method of arbitrarily adjusting a scanning angle of scan lines may be adopted to vary the viewing angle of the ultrasound image, instead of setting the scan lines to be perpendicular to the respective transducer elements. However, this scanning method is also limited in improving resolution of the ultrasound image.

### BRIEF DESCRIPTION OF THE DRAWINGS

FIG. 1 is a schematic diagram showing a conventional ultrasound probe adopted in the ultrasound system.

FIG. 2 is a schematic diagram showing scan lines set by a linear array transducer.

FIG. 3 is a schematic diagram showing scan lines set by a convex array transducer.

FIG. 4 is a schematic diagram showing an ultrasound probe in accordance with one embodiment of the present invention.

FIG. 5 is cross-sectional view schematically showing a geometrical structure of the array transducer in accordance with one embodiment of the present invention.

FIG. 6 is a schematic diagram showing an example of scan lines set by an array transducer in accordance with one embodiment of the present invention.

### DETAILED DESCRIPTION OF THE INVENTION

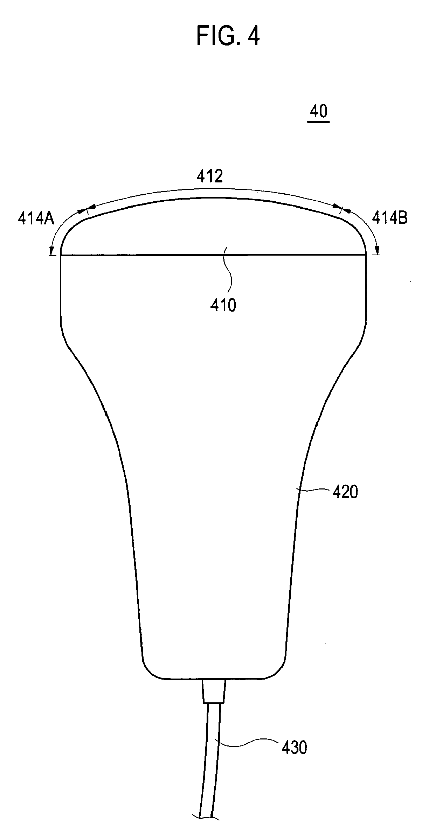

FIG. 4 is a schematic diagram showing an ultrasound probe, which is adopted in an ultrasound system in accordance with the present invention. The ultrasound probe 40 may include a transmission/reception (T/R) unit 410 and a body 420. The T/R unit 410 may include a curved array transducer for reciprocally converting ultrasound signals and electrical signals. The T/R unit 410 may further include at least one matching layer, a lens and a backing layer. The matching layer may be operable to reduce an acoustic impedance difference between the array transducer and a target object. The lens may be operable to focus the ultrasound signals on a specific region. The backing layer may be operable to shield the ultrasound signals propagated backward the curved array transducer. The body 420 may be designed to be easily held by the user. The reference numeral 430, which is denoted in FIG. 1, may be a cable connecting the probe 40 to a main body of the ultrasound system.

A surface of the T/R unit 410 for transmitting and receiving ultrasound signals may be formed in conformance to a shape of the array transducer in accordance with the present invention. That is, surface curvature of the T/R unit 410 may have the same curvature with the array transducer.

As illustrated in FIG. 4, the surface of the T/R unit 410 may be formed with a first portion 412, a second portion 414A and a third portion 414B. The first portion 412 may be positioned at a center portion of the surface of the T/R unit 410. A radius of curvature of the first portion 412 may be larger than those of the second and third portions 414A and 414B. The radii of the second and third portions 414A and 414B may be identical to each other in accordance with one embodiment of the present invention.

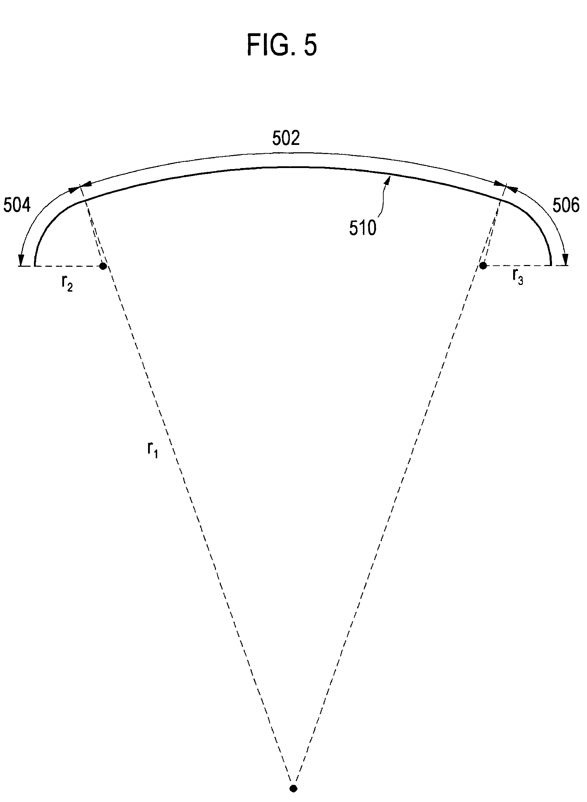

FIG. 5 is cross-sectional view schematically showing a structure of the curved array transducer mounted on the ultrasound probe, which is in accordance with one embodiment of the present invention. As illustrated in FIG. 5, the array transducer 510 may be formed with a first array unit 502, a second array unit 504 and a third array unit 506. The radii of curvature of the first to third array units 502-506 may be r₁, r₂ and r₃, respectively.

The first radius of curvature r₁ may be larger than radii of curvature r₂ and r₃. The first radius of curvature r₁ may be set so as to be close to infinity, i.e., the first array unit 502 may be formed in a linear shape. The radii of curvature r₂ and r₃ may be relatively small. That is, the radii of curvature r₂ and r₃ may be formed in a round shape. Thus, a high resolution ultrasound image may be obtained in a depth direction through the first array unit 502. Also, a wide viewing angle of ultrasound image may be obtained through the second and third array units 504 and 506. The second radius of curvature r₂ may be substantially identical to the third radius of curvature r₃ in accordance with one embodiment of the present invention. A width of the first array unit 502 may be relatively wider than those of the second and third array units 504 and 506. It is preferable that a ratio of the first array unit 502 to the array transducer 510 may be within a width range of about 50 % to 80 %.

Although the ultrasound probe has been explained as including an array transducer constructed with three array units in accordance with one embodiment of the present invention, the number of array units is certainly not limited thereto. The array transducer may include more than 3 array units, each having a different radius of curvature, in accordance with another embodiment of the present invention.

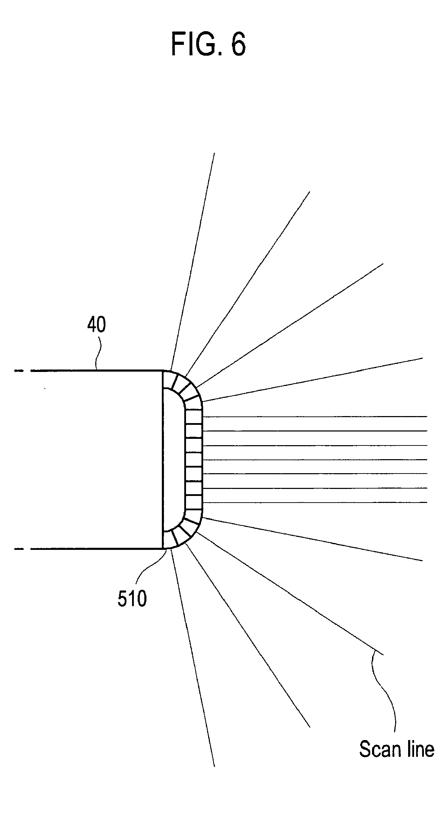

FIG. 6 is a schematic diagram showing an example of scan lines set by an array transducer 510 in accordance with one embodiment of the present invention. As illustrated in FIG. 6, the scan lines positioned at a center portion of the T/R unit are set at a uniform interval toward a depth direction while the scan lines positioned at both edge portions of the T/R unit are set in a radial shape. Thus, a high resolution ultrasound image may be obtained through the array unit positioned at a center portion of the T/R unit. Also, an ultrasound image wide of the viewing angle may be obtained through the array units positioned at the both edge portions of the T/R unit 410.

As mentioned above, the high resolution ultrasound image may be obtained through the center array having a relatively large radius of curvature. Also, the ultrasound image of the wide viewing angle may be obtained through the edge arrays having a relatively small radius of curvature.

In accordance with one embodiment of the present invention, there is provided an ultrasound probe in an ultrasound system, comprising: a body; and a transmission/reception unit mounted on the body and having at least one partially curved array transducer operable to reciprocally convert ultrasound signals and electric signals, wherein the array transducer is sized and configured to have multiple radii of curvature.

Any reference in this specification to "one embodiment," "an embodiment," "example embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound probe in an ultrasound system, comprising:
a body; and
a transmission/reception unit mounted on the body and having at least one partially curved array transducer operable to reciprocally convert ultrasound signals and electric signals,
wherein the curved array transducer is sized and configured to have multiple radii of curvature.

2. The ultrasound probe of Claim 1, wherein the curved array transducer includes:
a first array unit positioned at a center portion of the array transducer and being configured to have a first radius of curvature;
a second array unit positioned at one edge portion of the array transducer and being configured to have a second radius of curvature; and
a third array unit positioned at the other edge portion of the array transducer and being configured to have a third radius of curvature.

3. The ultrasound probe of Claim 2, wherein the first radius of curvature is greater than the second and third radii of curvature.

4. The ultrasound probe of Claim 3, wherein the second radius of curvature is substantially identical to the third radius of curvature.

5. The ultrasound probe of Claim 4, wherein the transmission/reception unit includes:
at least one matching layer operable to reduce an acoustic impedance difference between the array transducer and the target object;
a lens operable to focus the ultrasound signals on a specific region; and
a backing layer operable to shield the ultrasound signals propagated backward the array transducer.

6. The ultrasound probe of Claim 1, wherein the curved array transducer includes:
a first array unit positioned at a center portion of the curved array transducer and being configured to have a first radius of curvature;
a second array unit positioned at one edge portion of the array transducer, wherein the second array is configured to have multiple radii of curvature less than the first radius of curvature; and
a third array positioned at the other edge portion of the array transducer, wherein the third array is configured to have multiple radii of curvature less than the first radius of curvature.
